# EUROPEAN PATENT APPLICATION

(11) **EP 4 285 895 A1**
(43) Date of publication of application: **06.12.2023**
(21) Application number: 22177334.4
(22) Date of filing: 03.06.2022
(51) Int. Cl.: A61K 9/48, A61K 31/4178, A61K 47/38, A61K 47/42, A61K 9/26, A61K 47/32

(54) **NITROFURANTOIN ORAL DOSAGE FORM**

(71) Applicant: Adalvo Limited, San Gwann, SGN 3000 (MT)
(72) Inventor: Kalmoua, Faysal, Oss (NL); Feitzinger, Martina, Mondsee (AT)
(74) Representative: Harber IP s.r.o.

(57) **Abstract**

The present invention provides an oral dosage form of nitrofurantoin which is a capsule containing:
- macrocrystalline nitrofurantoin in an immediate release part, and
- nitrofurantoin monohydrate in a sustained-release part,
wherein the sustained-release part is in the form of tablets.

## Description

### Field of Art

The present invention relates to a pharmaceutical capsule formulation containing Nitrofurantoin immediate release blend and extended-release tablets.

### Background Art

Nitrofurantoin is an antibacterial agent specific for urinary tract infections. The brand product Macrobid^{®} is a hard gelatin capsule containing an equivalent of 100 mg of nitrofurantoin composed of 25 mg of nitrofurantoin macrocrystals and 75 mg of nitrofurantoin monohydrate in the form of a powder blend.

The chemical name of nitrofurantoin is 1-[[[5-nitro-2-furanyl]methylene]amino]-2,4-imidazolidinedione. The chemical structure is the following:

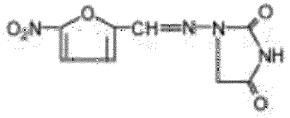

The chemical name of nitrofurantoin monohydrate is 1-[[[5-nitro-2-furanyl]methylene]amino]-2,4-imidazolidinedione monohydrate. The chemical structure is the following:

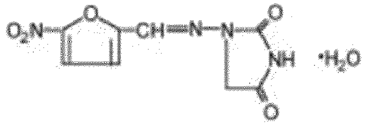

Macrocrystalline nitrofurantoin has slower dissolution and absorption than nitrofurantoin monohydrate. Nitrofurantoin monohydrate in the form of a powder blend, upon exposure to gastric and intestinal fluids, forms a gel matrix that releases nitrofurantoin over time. Nitrofurantoin monohydrate is very slightly soluble in water.

**Solubility of Nitrofurantoin Monohydrate and Macrocrystals in Different Media**

| **Medium** | **Monohydrate Solubility (mg/mL)** | **Macrocrystals Solubility (mg/mL)** |
|---|---|---|
| 0.1N HCl | 0.12 | 0.14 |
| 0.01N HCl | 0.13 | 0.14 |
| pH 4.5 Acetate Buffer | 0.04 | 0.05 |
| pH 6.8 Phosphate Buffer | 0.19 | 0.20 |
| pH 7.5 Phosphate Buffer | 0.55 | 0.62 |
| Water | 0.15 | 0.16 |

| | | |
|---|---|---|
| *Reference:* Notebook # ARD461, p.26-38, p.62-66 | | |

EP250023 and EP250038 describe a pharmaceutical capsule formulation containing Nitrofurantoin for oral administration comprising a particulate mixture in a capsule shell which is soluble in gastrointestinal juice, and wherein the first layer contains nitrofurantoin monohydrate in the form of a sustained release particulate mixture, and the second layer contains macrocrystalline nitrofurantoin in the form of a rapid release particulate mixture. A preferred process for producing the sustained release pharmaceutical capsules comprises the steps of: (1) preparing the particulate mixture, and (2) filling the particulate mixture into a capsule shell.

There is still a need to develop pharmaceutical formulations containing Nitrofurantoin with improved content uniformity, having the release profile similar to that of the reference drug, and suitable stability.

### Description of the invention

The present invention relates to an oral dosage form which is a sustained-release pharmaceutical capsule formulation containing a combination of immediate release and sustained-release Nitrofurantoin.

The oral dosage form which is a sustained release capsule contains:
- macrocrystalline nitrofurantoin in an immediate release part, and
- nitrofurantoin monohydrate in a sustained-release part,
wherein the sustained-release part is in the form of tablets.

The immediate release part is in the form of a powder blend containing macrocrystalline Nitrofurantoin and one or more pharmaceutically acceptable excipients selected from the group of a filler, a glidant and a first diluent, and optionally a disintegrant.

Particle size distribution of macrocrystalline Nitrofurantoin analyzed by air jet sieving (using Alpine Air Jet Sieve System) is as follows:
at least 20% w/w of the particles are greater than 250 µm,
at least 60% w/w of the particles are greater than 212 µm,
at least 85% w/w of the particles are greater than 125 µm.

The glidant is preferably selected from a group of talc, silica, magnesium carbonate, silicon dioxide. A particularly preferred glidant for the immediate release part is talc.

The filler is preferably selected from a group of corn starch, microcrystalline cellulose, mannitol, calcium phosphate. A particularly preferred filler is corn starch.

The disintegrant is preferably selected from a group of crospovidone, croscarmellose sodium, sodium starch glycolate, cross-linked alginic acid.

The first diluent is preferably selected from a group of lactose, dextrose, calcium phosphate, mannitol, starch, sorbitol, sucrose. A particularly preferred diluent for the immediate release part is lactose monohydrate or lactose.

Preferably, the immediate release part contains macrocrystalline nitrofurantoin, corn starch, talc and lactose monohydrate.

The immediate release part is prepared by blending the active substance with the one or more pharmaceutically acceptable excipients.

The sustained release part is in the form of tablets containing Nitrofurantoin monohydrate, a release modifier and one or more additional pharmaceutically acceptable excipients selected from the group of a release modifier, a binder, a second diluent, a glidant and a lubricant.

The largest dimension of tablets is up to 7 mm, preferably within the range of 4.5-6.5 mm. In some embodiments, the tablets might be cylindrical, with a diameter of 4.5-6.5 mm and height of 2.5-3.5 mm.

Particle size distribution of Nitrofurantoin Monohydrate analyzed by USP method by laser diffraction (using Sympatec HELOS/BF LASER Diffraction Spectrophotometer with VIBRI air injection and RODOS/M dry dispersing module (with Nilfisk Advance HEPA Vacuum)) is as follows:
D10 NMT (NMT = must not be more than) 10 µm, preferably NMT 8 µm,
D50 NMT 6-35 µm, preferably NMT 20-35 µm,
D90 NMT 120 µm, preferably NMT 100 µm.

The release modifier is preferably selected from a group of polymers like carbomer homopolymer, pectin, hydroxypropyl methylcellulose (HPMC), polyethylene glycol, polyethylene oxide. A particularly preferred release modifier is carbomer homopolymer (e.g., Carbopol 971-P, an acrylic acid crosslinked with allyl sucrose or allyl pentaerythritol).

The binder is preferably selected from a group of polyvinylpyrrolidone, starch, pregelatinized starch, hydroxypropyl cellulose, hydroxypropyl methylcellulose (HPMC). A particularly preferred binder is polyvinylpyrrolidone (povidone).

The second diluent is preferably selected from a group of powdered sugars like confectioners' sugar, lactose, mannitol, microcrystalline cellulose. A particuarly preferred second diluent for the sustaned release part is confectioners' sugar.

The glidant is preferably selected from a group of talc, silica, magnesium carbonate, silicon dioxide. A particularly preferred glidant is talc.

The lubricant is magnesium stearate.

Preferably, the sustained release tablets contain Nitrofurantoin Monohydrate, carbomer homopolymer, polyvinylpyrrolidone, confectioners' sugar, talc and magnesium stearate.

Tablets are prepared by wet granulation process using a granulation liquid based on water, ethanol and/or a mixture thereof. In a preferred embodiment of the invention the granulation liquid is a solution of the binder in a mixture of water and ethanol, wherein preferably the ratio of water and ethanol is 80: 20 w/w.

In some embodiments, the oral dosage form according to the invention contains:
a. an immediate release blend containing:
   15 - 25 % w/w of Nitrofurantoin macrocrystals,
   5 - 15 % w/w of glidant,
   10 - 15 % w/w of filler,
   55 - 65 % w/w of diluent,
   wherein the % is relative to the weight of the immediate release blend; and
b. sustained release tablets containing:
   40 - 50 % w/w of Nitrofurantoin monohydrate,
   3 - 5 % w/w of release modifier,
   25 - 30 % w/w of binder,
   15 - 25 % w/w of diluent,
   3 - 5 % w/w of glidant and lubricant,
   wherein the % is relative to the weight of the sustained release tablets.

In some embodiments, the oral dosage form according to the invention contains
a. an immediate release blend containing:
   15 - 25 % w/w of Nitrofurantoin macrocrystals,
   5 - 15 % w/w of talc,
   10 - 15 % w/w of corn starch,
   55 - 65 % w/w of lactose monohydrate,
   wherein the % is relative to the weight of the immediate release blend; and
b. sustained release tablets containing:
   40 - 50 % w/w of Nitrofurantoin monohydrate,
   3 - 5 % w/w of carbomer homopolymer,
   25 - 30 % w/w of povidone,
   15 - 25 % w/w of confectioner's sugar,
   1.5 - 2.5 % w/w of talc,
   1.5 - 2.5 % w/w of magnesium stearate,
   wherein the % is relative to the weight of the sustained release tablets.

The immediate release blend and the sustained release tablets are combined in the ratio corresponding to the amount of the immediate release blend equivalent to 1 weight part of Nitrofurantoin and the amount of the sustained release tablets equivalent to 3 weight parts of Nitrofurantoin.

The immediate release blend and the sustained release tablets are combined in the ratio corresponding to the amount of the immediate release blend equivalent to 25 mg of Nitrofurantoin and the amount of the sustained release minitablets equivalent to 75 mg of Nitrofurantoin.

More preferably, the oral dosage form according to the invention contains:
a. an immediate release blend containing:
   15 - 25 % w/w of Nitrofurantoin macrocrystals,
   5 - 15 % w/w of talc,
   10 - 15 % w/w of corn starch,
   55 - 65 % w/w of lactose monohydrate,
   wherein the % is relative to the weight of the immediate release blend; and
b. sustained release tablets or tablets containing
b1. an intragranular mixture containing:
   40 - 50 % w/w of Nitrofurantoin monohydrate,
   3 - 5 % w/w of carbomer homopolymer,
   25 - 30 % w/w of povidone,
   15 - 25 % w/w of confectioner's sugar; and
b2. an extragranular phase containing:
   1.5 - 2.5 % w/w of talc,
   1.5 - 2.5 % w/w of magnesium stearate,
   wherein the % is relative to the weight of the sustained release tablets;
filled in hard gelatine capsule or HPMC capsule,
wherein the immediate release blend and the sustained release tablets are combined in the ratio corresponding to the amount of the immediate release blend equivalent to 1 weight part of Nitrofurantoin and the amount of the sustained release tablets equivalent to 3 weight parts of Nitrofurantoin.

Yet more preferably, the oral dosage form according to the invention contains
a. an immediate release blend containing:
   18 - 22 % w/w of Nitrofurantoin macrocrystals,
   8 - 12 % w/w of talc,
   11 - 14 % w/w of corn starch,
   56 - 60 % w/w of lactose monohydrate,
   wherein the % is relative to the weight of the immediate release blend; and
b. sustained release tablets or tablets containing
b1. an intragranular mixture containing:
   43 - 47 % w/w of Nitrofurantoin monohydrate,
   3 - 5 % w/w of carbomer homopolymer,
   26 - 29 % w/w of povidone,
   17 - 22 % w/w of confectioner's sugar; and
b2. an extragranular phase containing:
   1.5 - 2.5 % w/w of talc,
   1.5 - 2.5 % w/w of magnesium stearate,
   wherein the % is relative to the weight of the sustained release tablets;
filled in hard gelatine capsule or HPMC capsule,
wherein the immediate release blend and the sustained release tablets are combined in the ratio corresponding to the amount of the immediate release blend equivalent to 1 weight part of Nitrofurantoin and the amount of the sustained release tablets equivalent to 3 weight parts of Nitrofurantoin.

In the most preferred embodiment of the invention the oral dosage form contains
a. an immediate release blend containing

| **Ingredients** | **mg** | **% w/w** |
|---|---|---|
| Nitrofurantoin Macrocrsytals, USP | 25.00 | 19.841 |
| Talc, USP (Imperial 1885L) | 11.50 | 9.127 |
| Corn Starch, NF (UNI-Pure^{®}F) | 16.00 | 12.698 |
| Lactose Monohydrate, NF (Pharma 100M) | 73.50 | 58.334 |
| 126.00 | | 100.000 |

b. sustained release tablets containing

| **Ingredients** | **mg/unit** (tablet) | **% W/W** |
|---|---|---|
| Nitrofurantoin Monohydrate, USP | 40.35 | 45.593 |
| Carbomer Homopolymer NF, (Type A) (Carbopol 971-P) | 3.38 | 3.814 |
| Povidone USP/NF (Plasdone K-29/32) | 24.00 | 27.119 |
| Confectioners' Sugar USP/NF | 17.28 | 19.520 |
| Purified Water, USP | q.s | q.s |
| Dehydrated Alcohol, USP | q.s | q.s |
| Talc USP (Imperial 1885L) | 1.75 | 1.977 |
| Magnesium Stearate, NF | 1.75 | 1.977 |
| | 88.5 | 100.000 |

wherein two tablets are combined with the immediate release blend corresponding to 25 mg of nitrofurantoin.

The above-described sustained release tablets and an immediate release powder blend are filled into capsule (preferably a hard gelatine capsule or HPMC capsule).

The capsules according to the present invention are capsules having a shell which is soluble in gastrointestinal juice. The capsules according to the present invention comprise hard gelatin capsules and HPMC capsules. The preferred capsules are hard gelatin capsules which are soluble in gastric juice.

The oral dosage form of the present invention preferably has the following in vitro release of Nitrofurantoin measured in 900 ml of 0.01N Hydrochloric acid during the first hour and subsequently measured in 950 ml USP buffer pH= 7.5 using USP Apparatus II (Paddles) with Sinkers at 100 rpm and 37 °C:
not more than 20 % of Nitrofurantoin released within 1 hour,
not less than 20 % and not more than 70 % of Nitrofurantoin released within 2 hours, and
at least 80 % of Nitrofurantoin released within 7 hours.

The matrix structure and shape of the oral dosage form play a critical role in having better control on the release rate of active substance from the extended-release matrix.

The structure and shape of a gel matrix produced from a powder varies when it gets in contact with intestinal fluids, and a plug of powder mass usually does not have a defined structure. Therefore, the release of the active substance from such matrix over a period of time shows high variation.

In contrast to that, the matrix in tablets is formed through well-defined parameters followed during manufacturing. The matrix structure of the tablets is reproducible, the shape thereof is defined, and therefore, the release of drug substance is more consistent in the release profiles as evident by lower RSD (Relative Standard Deviation) from the tablet formulation than the powder formulation. Sustained-release tablets have the following advantages: improved content uniformity, consistent and reproducible dissolution rate, and improved product stability.

Furthermore, the variation in the weight of the mass in tablets is lower than the blend, when filled in the capsule form.

### Description of drawings

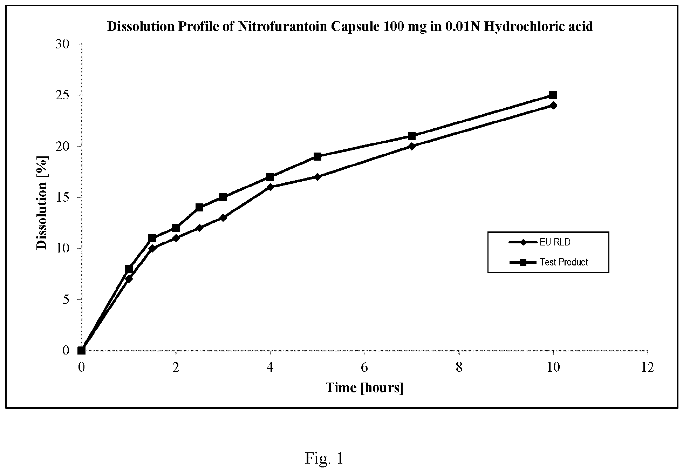

Fig. 1 is a graphical comparison of dissolution profiles of 100 mg Nitrofurantoin EU reference product Macrobid^{®} and the solid oral dosage form according to the invention in 0.01 N Hydrochloric acid dissolution medium

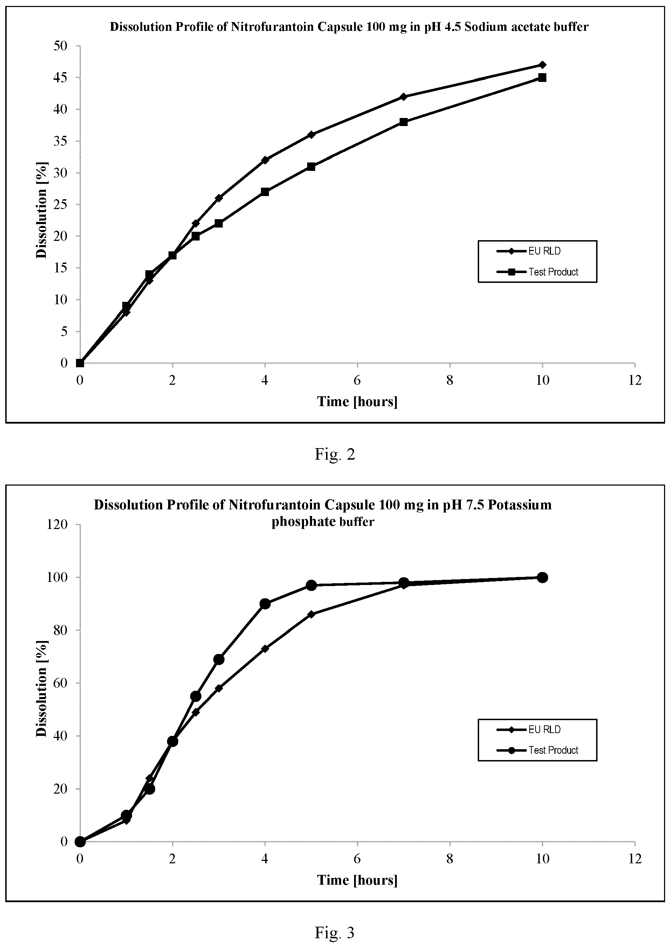

Fig. 2 is a graphical comparison of dissolution profiles of 100 mg Nitrofurantoin EU reference product Macrobid^{®} and the solid oral dosage form according to the invention in pH 4.5 Sodium acetate buffer dissolution medium
Fig. 3 is a graphical comparison of dissolution profiles of 100 mg Nitrofurantoin EU reference product Macrobid^{®} and the solid oral dosage form according to the invention in pH 7.5 Potassium phosphate buffer dissolution medium

### Example of carrying out the Invention

**Immediate release Nitrofurantoin Macrocrystals Blend**

| **Name of Ingredients** | **Function** | **Reference to Standard** | **Formula** | |
|---|---|---|---|---|
| | | | **mg/unit** | **% W/W** |
| Nitrofurantoin Macrocrystals, USP | API | USP | 25.00 | 19.841 |
| Talc, USP (Imperial 1885L) | Glidant | USP | 11.50 | 9.127 |
| Corn Starch, NF (UNI-Pure^{®} F) | Filler | NF | 16.00 | 12.698 |
| Lactose Monohydrate, NF (Pharma 100M) | Diluent | NF | 73.50 | 58.334 |
| Total | | | 126.00 | 100.000 |

The immediate release powder blend containing Nitrofurantoin Macrocrystals is prepared using the following procedure:
- Load Nitrofurantoin macrocrystals, Starch and Talc in a V-shell blender and perform blending to obtain a pre-blend.
- Unload the pre-blend and pass through a sieve to break agglomerate.
- Divide pre-blend into three equal parts.
- Divide Lactose monohydrate into three equal parts.
- Load pre-blend and Lactose monohydrate in the following order in a V-shell blender and perform blending:
   - Lactose monohydrate Part-1
   - Sieved Pre-blend Part-1
   - Lactose monohydrate Part-2
   - Sieved Pre-blend Part-2
   - Lactose monohydrate Part-3
   - Sieved Pre-blend Part-3
- Unload blend and pass through a sieve which is a 20-mesh screen.
- Load the sieved blend in V-shell blender and blend it.
- Unload blend in a container.

**Sustained release Nitrofurantoin monohydrate tablets:**

| **Material Description** | **Function** | **Reference to Standard** | **Formula** | |
|---|---|---|---|---|
| | | | **mg/unit (Tablet)** | **% W/W** |
| **Intra-granular part** | | | | |
| Nitrofurantoin Monohydrate, USP | API | USP | 40.35 | 45.593 |
| Carbomer Homopolymer NF, (Type A) (Carbopol 971-P) | Release modifier | NF | 3.38 | 3.814 |
| Povidone USP/NF (Plasdone K-29/32) | Binder | USP/NF | 24.00 | 27.119 |
| Confectioners' Sugar USP/NF | Diluent | USP/NF | 17.28 | 19.520 |
| Purified Water, USP | Solvent | USP | q.s | q.s |
| Dehydrated Alcohol, USP | Solvent | USP | q.s | q.s |

| **Extra-granular part** | | | | |
|---|---|---|---|---|
| Talc USP (Imperial 1885L) | Glidant | USP | 1.75 | 1.977 |
| Magnesium Stearate, NF | Lubricant | NF | 1.75 | 1.977 |
| **Sub- Total:** | | | 88.50 | 100.000 |

Sustained release tablets containing Nitrofurantoin monohydrate are prepared by the following procedure:
- Load Nitrofurantoin Monohydrate, Carbomer 974P, Povidone K-29/32 and confectioner's sugar 6X in V-Shell blender and perform blending.
- Unload the blend and pass through a Comil.
- Load the milled blend in a V-shell blender and perform blending.
- Unload the blend and pass through a Comil.
- Load the milled blend in a V-shell blender and perform blending.
- Transfer the blend in a bowl of top-spray fluid bed processor.
- Perform granulation using 80:20 water-ethanol binder solution
- Pass the granules through a Comil.
- Load milled granules and talc in a V-shell blender and perform blending
- Add magnesium stearate and perform final blending.
- Compress the blended granules into tablet, which is equivalent to 37.5 mg of Nitrofurantoin, using a tablet press.

Capsules containing 100 mg of Nitrofurantoin in form of Nitrofurantoin Monohydrate and Nitrofurantoin Macrocrystals are as follows:

| **Item** | **Material Description** | | **mg/capsule** |
|---|---|---|---|
| 1 | Nitrofurantoin Monohydrate Tablets, 37.5 mg (sustained release tablets) | | 2 x 88.5 mg (2 Tablets) |
| 2 | Nitrofurantoin Macrocrystals Final Blend, 25 mg (immediate release blend) | | 126 |
| 3 | Hard Gelatin Capsules, Size 1 Coni-Snap White Cap / White Opaque Body | | 76 (1 cap) |
| **Total Net Weight** | | | 303 |
| **Total Weight of Filled Capsule** | | | 379 |

### Capsule filling:

Fill the immediate release blend equivalent to 25mg of Nitrofurantoin and 2 Nitrofurantoin monohydrate tablets equivalent to 75 mg of Nitrofurantoin, in a hard gelatin capsule.

The reference product Macrobid^{®} and the sustained release capsules according to the invention were tested for dissolution according to the below method.

**Dissulution procedure in different media**

| **Dissolution media** | | |
|---|---|---|
| **0.01 N HCl** | **Buffer pH 4.5** | **Buffer pH 7.5** |
| All specification time points in 900 mL of 0.01 N HCl | 900 mL of 0.01 N HCl solution [5.1.2.1] added. After 1 hour time point 50 mL of Acetate Buffer Concentrate [5.1.2.5] added and continued to remaining time points with pH 4.5 dissolution medium | 900 mL of 0.01 N HCl solution [5.1.2.1] added. After 1 hour time point 50 mL of Phosphate Buffer concentrate [5.1.2.2] added and continued to remaining time points with pH 7.5 dissolution medium |

**Medium and conditions for performing the dissolution test**

| Apparatus | USP Apparatus II (Paddles) with Sinkers |
|---|---|
| Speed | 100 rpm |
| | 900 mL for 0.01 N HCl; |
| Medium Volume | 950 mL for Buffer pH 4.5; |
| | 950 mL for Buffer pH 7.5 |
| Sampling time | 1, 1.5, 2, 2.5, 3, 4, 5, 7 and 10 hours |
| Collection volume | Autosampler: 1.5 mL Manual sampling: 10 mL |
| Temperature of the medium | (37±0.5)°C |

**Stability data results of test product at long term storage conditions: (25±2)°C/ (60±5)% RH (150cc wide mouth round HDPE bottle, 38-400 CR (Child Resistant) Finish)**

| **TESTS** | | | **TIME POINT, MONTHS** | | | |
|---|---|---|---|---|---|---|
| | | | **0** | **1** | **3** | **6** |
| **Appearance** | | | | | | |
| Gelatin capsules | | | Conforms | Conforms | Conforms | Conforms |
| **Assay** | | | | | | |
| 95.0% - 105.0% of label claim | | | 101.6% | 99.7 % | 99.4% | 98.7% |
| **Related Compounds-1** | | | | | | |
| N-(aminocarbonyl)-N-[([5-nitro-2-furanyl] methylene) amino] glycine - NMT 0.2% | | | Not detected | Not detected | Not detected | Not detected |
| Any Unspecified impurity - NMT 0.2% | | | Not detected | BQL | BQL | BQL |
| **Related Compounds-2** | | | | | | |
| 5-Nitro-2-Furoic Acid - NMT 0.2% | | | Not detected | Not detected | Not detected | Not detected |
| 5-Nitro-2-Furaldehyde - NMT 0.2% | | | Not detected | Not detected | Not detected | Not detected |
| **Total Related Compounds** | | | | | | |
| Total Related Compounds (RC-1 + RC-2) - NMT 1.5% | | | Not detected | BQL | BQL | BQL |
| **Limit of Nitrofurazone** | | | | | | |
| NMT 0.01% | | | Not detected | Not detected | Not detected | Not detected |
| **Dissolution** | | | | | | |
| 1 hour | Mean value NMT 20% | Min (%) | 5 | 5 | 5 | 5 |
| | | Max (%) | 11 | 9 | 9 | 8 |
| | | AVG (%) | 7 | 7 | 6 | 6 |
| | | RSD (%) | 21.9 | 18.7 | 19.4 | 13.0 |
| 2 hours | Mean value 25% - 65% | Min (%) | 24 | 36 | 30 | 33 |
| | | Max (%) | 44 | 47 | 47 | 44 |
| | | AVG (%) | 38 | 41 | 38 | 40 |
| | | RSD (%) | 10.6 | 9.0 | 14.1 | 9.2 |
| 7 hours | Mean value | Min (%) | 94 | 94 | 93 | 98 |
| | NLT 80% | Max (%) | 101 | 102 | 101 | 102 |
| | | AVG (%) | 98 | 97 | 97 | 100 |
| | | RSD (%) | 2.1 | 2.2 | 2.6 | 1.4 |
| **Water content** | | | | | | |
| NMT 9.0% | | | 4.9 | 5.2 | 5.7 | 6.1 |

| | | | | | | |
|---|---|---|---|---|---|---|
| BQL - Below Quantitation Limit NMT - No More Than RSD - Relative Standard Deviation | | | | | | |

**Stability data results of test product at accelerated storage conditions: (40±2)°C/ (75±5)% RH (150cc wide mouth round HDPE bottle, 38-400 CR Finish)**

| **TESTS** | | | | **TIME POINT, MONTHS** | | | | |
|---|---|---|---|---|---|---|---|---|
| | | | | **0** | **1** | **2** | **3** | **6** |
| | | **Assay** | | | | | | |
| 95.0% - 105.0% of label claim | | | | 101.6 | 99.6 | 101.1 | 98.1 | 99.2 |
| | | **Related Compounds-1** | | | | | | |
| N-(aminocarbonyl)-N- [([5-nitro-2-furanyl] methylene) amino] glycine - NMT 0.2% | | | | Not detected | Not detected | Not detected | Not detected | Not detected |
| Any Unspecified impurity - NMT 0.2% | | | | Not detected | BQL | Not detected | BQL | BQL |
| | | **Related Compounds-2** | | | | | | |
| 5-Nitro-2-Furoic Acid - NMT 0.2% | | | | Not detected | Not detected | Not detected | Not detected | Not detected |
| 5-Nitro-2-Furaldehyde - NMT 0.2% | | | | Not detected | Not detected | BQL | BQL | Not detected |
| | | **Total Related Compounds** | | | | | | |
| Total Related Compounds (RC-1 + RC-2) - NMT 1.5% | | | | Not detected | BQL | BQL | BQL | BQL |
| | | **Limit of Nitrofurazone** | | | | | | |
| NMT 0.01% | | | | Not detected | Not detected | Not detected | Not detected | Not detected |
| | | **Dissolution** | | | | | | |
| 1 hour | Mean value NMT 20% | | Min (%) | 5 | 6 | 5 | 5 | 5 |
| | | | Max (%) | 11 | 10 | 10 | 8 | 12 |
| | | | AVG (%) | 7 | 8 | 7 | 6 | 7 |
| | | | RSD (%) | 19.89 | 17.06 | 21.31 | 15.55 | 27.16 |
| 2 hours | Mean value 25% - 65% | | Min (%) | 24 | 34 | 38 | 38 | 54 |
| | | | Max (%) | 44 | 54 | 57 | 57 | 64 |
| | | | AVG (%) | 37 | 45 | 49 | 46 | 59 |
| | | | RSD (%) | 14.23 | 10.56 | 12.52 | 9.16 | 4.96 |
| 7 hours | Mean value NLT 80% | | Min (%) | 94 | 95 | 95 | 95 | 98 |
| | | | Max (%) | 102 | 101 | 102 | 100 | 102 |
| | | | AVG (%) | 97 | 99 | 99 | 97 | 100 |
| | | | RSD (%) | 2.02 | 1.85 | 2.13 | 1.48 | 1.42 |
| | | **Water content** | | | | | | |
| NMT 9.0% | | | | 4.9 | 5.3 | 5.2 | 5.8 | 6.2 |

| | | | | | | | | |
|---|---|---|---|---|---|---|---|---|
| BQL - Below Quantitation Limit NMT - No More Than RSD - Relative Standard Deviation | | | | | | | | |

## Claims

1. An oral dosage form which is a capsule containing:
- macrocrystalline nitrofurantoin in an immediate release part, and
- nitrofurantoin monohydrate in a sustained-release part,
wherein the sustained-release part is in the form of tablets.

2. The oral dosage form according to claim 1 which has the following in vitro release of Nitrofurantoin measured in 900 ml of 0.01N Hydrochloric acid during the first hour and subsequently measured in 950 ml USP buffer pH= 7.5 using USP Apparatus II (Paddles) with Sinkers at 100 rpm and 37 °C:
not more than 20 % of Nitrofurantoin released within 1 hour,
not less than 20 % and not more than 70 % of Nitrofurantoin released within 2 hours, and
at least 80 % of Nitrofurantoin released within 7 hours.

3. The oral dosage form according to any one of the preceding claims, wherein the immediate release part is in the form of a powder blend containing macrocrystalline Nitrofurantoin and one or more pharmaceutically acceptable excipients selected from the group of a filler, a glidant and a first diluent, and optionally a disintegrant.

4. The oral dosage form according to any one of the preceding claims, wherein the immediate release part contains macrocrystalline nitrofurantoin, corn starch, talc and lactose monohydrate.

5. The oral dosage form according to any one of the preceding claims, wherein the sustained release part is in the form of tablets containing Nitrofurantoin monohydrate, a release modifier and one or more additional pharmaceutically acceptable excipients selected from the group of a release modifier, a binder, a second diluent, a glidant and a lubricant.

6. The oral dosage form according to any one of the preceding claims, wherein the largest dimension of tablets is up to 7 mm, preferably within the range of 4.5-6.5 mm.

7. The oral dosage form according to any one of the preceding claims, wherein the tablets are cylindrical, with a diameter of 4.5-6.5 mm and height of 2.5-3.5 mm.

8. The oral dosage form according to any one of the preceding claims, wherein the sustained release part is in the form of tablets containing Nitrofurantoin monohydrate, carbomer homopolymer, polyvinylpyrrolidone, confectioners' sugar, talc and magnesium stearate.

9. The oral dosage form according to claim 1 or 2, which contains:
a. an immediate release blend containing:
15 - 25 % w/w of Nitrofurantoin macrocrystals,
5 - 15 % w/w of glidant,
10 - 15 % w/w of filler,
55 - 65 % w/w of diluent,
wherein the % is relative to the weight of the immediate release blend; and
b. sustained release tablets containing:
40 - 50 % w/w of Nitrofurantoin monohydrate,
3 - 5 % w/w of release modifier,
25 - 30 % w/w of binder,
15 - 25 % w/w of diluent,
3 - 5 % w/w of glidant and lubricant,
wherein the % is relative to the weight of the sustained release tablets.

10. The oral dosage form according to claim 1 or 2, which contains:
a. an immediate release blend containing:
15 - 25 % w/w of Nitrofurantoin macrocrystals,
5 - 15 % w/w of talc,
10 - 15 % w/w of corn starch,
55 - 65 % w/w of lactose monohydrate,
wherein the % is relative to the weight of the immediate release blend; and
b. sustained release tablets containing:
40 - 50 % w/w of Nitrofurantoin monohydrate,
3 - 5 % w/w of carbomer homopolymer,
25 - 30 % w/w of povidone,
15 - 25 % w/w of confectioner's sugar,
1.5 - 2.5 % w/w of talc,
1.5 - 2.5 % w/w of magnesium stearate,
wherein the % is relative to the weight of the sustained release tablets.

11. The oral dosage form according to any one of the preceding claims, wherein the immediate release blend and the sustained release tablets are combined in the ratio corresponding to the amount of the immediate release blend equivalent to 1 weight part of Nitrofurantoin and the amount of the sustained release tablets equivalent to 3 weight parts of Nitrofurantoin.

12. The oral dosage form according to any one of the preceding claims, wherein the immediate release blend and the sustained release tablets are combined in the ratio corresponding to the amount of the immediate release blend equivalent to 25 mg of Nitrofurantoin and the amount of the sustained release minitablets equivalent to 75 mg of Nitrofurantoin.

13. The oral dosage form according to claim 1 or 2, which contains:
a. an immediate release blend containing:
15 - 25 % w/w of Nitrofurantoin macrocrystals,
5 - 15 % w/w of talc,
10 - 15 % w/w of corn starch,
55 - 65 % w/w of lactose monohydrate,
wherein the % is relative to the weight of the immediate release blend; and
b. sustained release tablets or tablets containing
b1. an intragranular mixture containing:
40 - 50 % w/w of Nitrofurantoin monohydrate,
3 - 5 % w/w of carbomer homopolymer,
25 - 30 % w/w of povidone,
15 - 25 % w/w of confectioner's sugar; and
b2. an extragranular phase containing:
1.5 - 2.5 % w/w of talc,
1.5 - 2.5 % w/w of magnesium stearate,
wherein the % is relative to the weight of the sustained release tablets;
filled in hard gelatine capsule or HPMC capsule,
wherein the immediate release blend and the sustained release tablets are combined in the ratio corresponding to the amount of the immediate release blend equivalent to 1 weight part of Nitrofurantoin and the amount of the sustained release tablets equivalent to 3 weight parts of Nitrofurantoin.

14. The oral dosage form according to claim 1 or 2, which contains:
a. an immediate release blend containing:
18 - 22 % w/w of Nitrofurantoin macrocrystals,
8 - 12 % w/w of talc,
11 - 14 % w/w of corn starch,
56 - 60 % w/w of lactose monohydrate,
wherein the % is relative to the weight of the immediate release blend; and
b. sustained release tablets or tablets containing
b1. an intragranular mixture containing:
43 - 47 % w/w of Nitrofurantoin monohydrate,
3 - 5 % w/w of carbomer homopolymer,
26 - 29 % w/w of povidone,
17 - 22 % w/w of confectioner's sugar; and
b2. an extragranular phase containing:
1.5 - 2.5 % w/w of talc,
1.5 - 2.5 % w/w of magnesium stearate,
wherein the % is relative to the weight of the sustained release tablets;
filled in hard gelatine capsule or HPMC capsule,
wherein the immediate release blend and the sustained release tablets are combined in the ratio corresponding to the amount of the immediate release blend equivalent to 1 weight part of Nitrofurantoin and the amount of the sustained release tablets equivalent to 3 weight parts of Nitrofurantoin.
